# EUROPÄISCHE PATENTSCHRIFT

(11) **EP 2 103 595 B1**
(45) Veröffentlichungstag und Bekanntmachung des Hinweises auf die Patenterteilung: **19.04.2017**
(21) Anmeldenummer: 09003730.0
(22) Anmeldetag: 16.03.2009
(51) Int. Cl.: C07C 209/78, C07C 263/10, C07C 211/50, C07C 265/14

(54) **Verfahren zur Herstellung von Di- und Polyaminen der Diphenylmethanreihe**
Method for producing di and polyamines of diphenylmethane series
Procédé destiné à la fabrication de di- et polyamines de la série diphénylméthane

(30) Priorität: 20.03.2008 DE 102008015123
(43) Veröffentlichungstag der Anmeldung: 23.09.2009
(73) Patentinhaber: Covestro Deutschland AG, 51373 Leverkusen (DE)
(72) Erfinder: Wershofen, Stefan, Dr., 41065 Mönchengladbach (DE); Müller, Heinz-Herbert, Dr., 47809 Krefeld (DE); Adamson, Richard, 42799 Leichlingen (DE); Pohl, Fritz, Dr., 25541 Brunsbüttel (DE); Sommer, Knut, Dr., 47804 Krefeld (DE)
(74) Vertreter: Levpat

(56) Entgegenhaltungen:
- WO-A-99/40059
- FLUKA CHEMIEKALIEN KATALOG: "Chemica Biochemika Analytika" 1997, SIGMA-ALDRICH HANDELS GMBH , WIEN , XP002532662 * Seite 146 *

## Beschreibung

Die Erfindung betrifft ein Verfahren zur Herstellung von Di- und Polyaminen der Diphenylmethanreihe (MDA) durch Umsetzung von Anilin und Formaldehyd in Gegenwart eines sauren Katalysators bei dem das eingesetzte Anilin in der Summe weniger als 0,5 Gew.%, bevorzugt 0,0001 bis 0,4 Ges.-%, besonders bevorzugt 0,0001 bis 0,3 Gew.-%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen.

Die Carbonylgruppen enthaltenden Verbindungen und/oder daraus durch Reaktion mit Anilin entstehenden Verbindungen können entweder dem frisch eingesetzten Anilin entstammen, sind im zurückgeführten Anilin enthalten oder werden im Laufe der Herstellung von Di- und Polyaminen der Diphenylmethanreihe als Nebenprodukte gebildet. Sie können ggf. im Anilinkreislauf angereichert werden und Gehalte erreichen, die über denjenigen im frischen Anilin liegen.

Unter Di- und Polyaminen der Diphenylmethanreihe (MDA) werden Amine und Gemische von Aminen folgenden Typs verstanden: 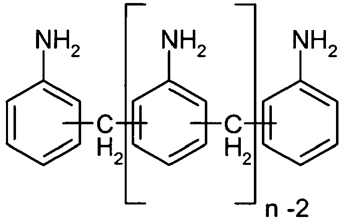 wobei n für eine natürliche Zahl ≥ 2 steht.

Für Verbindungen und Verbindungsgemische mit n = 2 sind dabei auch die Bezeichnung monomeres MDA (MMDA) und für Verbindungen und Verbindungsgemische mit n > 2 auch die Bezeichnung polymeres MDA (PMDA) üblich. Verbindungsgemische, in denen Verbindungen mit n = 2 und n > 2 nebeneinander vorkommen, werden üblicherweise vereinfachend unter dem Begriff MDA (Di- und Polyamine der Diphenylmethanreihe) zusammengefasst.

Die kontinuierliche, diskontinuierliche oder halbkontinuierliche Herstellung von Di- und Polyaminen der Diphenylmethanreihe ist in zahlreichen Publikationen und Patenten beschrieben (beispielsweise H.J. Twitchett, Chem. Soc. Rev. 3(2), 209 (1974); M.V. Moore in : Kirk-Othmer Encycl. Chem. Technol., 3rd Ed., New York, 2, 338-348 (1978); EP-A-31 423; EP-B-1 167 343; EP-A-1 403 242; EP 934 922 B1, WO 99/40059).
Üblicherweise erfolgt bei den technisch angewandten Verfahren die Herstellung von MDA durch Umsetzung von Anilin und Formaldehyd in Anwesenheit saurer Katalysatoren, wobei zum Ende des Prozesses der saure Katalysator üblicherweise durch Zugabe einer Base neutralisiert, das Reaktionsgemisch in eine organische und eine wässrige Phase getrennt und die organische Phase den abschließenden Aufarbeitungsschritten, wie beispielsweise der destillativen Entfernung von überschüssigem Anilin, zugeführt wird (US-A-5,310,769; DE-A-198 04 918; JP-A-2004026753).
Allen in der Literatur beschriebenen Verfahren zur Herstellung von MDA durch Umsetzung von Anilin und Formaldehyd in Gegenwart eines sauren Katalysators ist gemeinsam, dass bei der Umsetzung Chromophore gebildet werden, die das erzeugte MDA verfärben. Diese Verfärbungen werden bei der Neutralisation des sauren Katalysators und der Entfernung des bei der Umsetzung im Überschuss eingesetzten Anilins nicht oder nur unzureichend vermindert bzw. entfernt und führen bei der anschließenden Phosgenierung des MDA zu den entsprechenden Di- und Polyisocyanaten der Diphenylmethanreihe und deren anschließenden Aufarbeitung (Abtrennung des Lösungsmittels, Abtrennung von monomerem MDI) oft zu dunkel gefärbten Produkten, die wiederum gelblich verfärbte Polyurethanschäume oder andere verfärbte Polyurethan (PUR)-Materialien ergeben. Obwohl die Eigenfarbe der Di- und Polyisocyanate der Diphenylmethanreihe die mechanischen Eigenschaften der daraus hergestellten Polyurethane nicht negativ beeinflusst, sind helle Produkte wegen deren guten Variabilität im Produktionsprozess des Verarbeiters, z.B. hinsichtlich Durchscheinen durch dünne Deckschichten und farbliche Gestaltungsmöglichkeiten, bevorzugt.
Es hat nicht an Versuchen gefehlt, die Verfärbungen des MDA und des daraus hergestellten MDI zu vermindern.
In EP 1 270 544 B1 wird ein Verfahren zur Herstellung von MDA unter Minimierung des Gehalts an unerwünschten Nebenprodukten durch die Umsetzung von Anilin mit Formaldehyd in Gegenwart saurer Katalysatoren beschrieben, dadurch gekennzeichnet, dass man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50% der gesamt einzuspeisenden Formaldehydmenge die Reaktionsmischung auf eine Temperatur von größer 75°C temperiert. Reklamiert wird speziell die Minimierung des Gehalts an N-Methyl-MDA, dessen Verminderung im MDA gemäß der Lehre des EP 1 270 544 B1 bei einer anschließenden Phosgenierung zu einem Roh-MDI einer helleren Farbe führen soll.

Die Verbesserung der Farbwerte durch eine Verringerung des Gehalts an N-Methyl-MDA im erzeugten MDA über eine spezielle Formaldehydspeisung liegt auch folgenden Verfahren zugrunde.

DD-A-295 628 beschreibt für ein diskontinuierliches Verfahren die Zugabe des Formaldehyds in zwei Schritten während der Kondensationsstufe, wobei in der ersten Zugabe die Hauptmenge des Formaldehyds bei niederer Temperatur erfolgt und die zweite Zugabe des restlichen Formaldehyds bei gleicher oder höherer Temperatur erfolgt.

EP-A-451 442 und DD-A-238 042 offenbaren für ein kontinuierliches Verfahren die Zugabe von Formaldehyd über mehrere Verfahrensstufen.

Zur Verbesserung der Farbwerte lehrt US-A-5 286 760 neben der Minimierung des Gehalts an N-Methyl-MDA zusätzlich die Minimierung der Nebenkomponenten Acridan und Acridin. US-A-5 286 760 modifiziert jedoch nicht die Formaldehydspeisung, sondern die der primären Umsetzung des Formaldehyds mit Anilin folgenden Umlagerungen. US-A-5 286 760 beschreibt für eine kontinuierliche MDA-Herstellung eine Teilneutralisierung des Reaktionsgemisches zwischen der Kondensationsstufe von zwei Molekülen Anilin und einem Molekül Formaldehyd und der anschließenden Umlagerung der intermediär gebildeten Aminobenzylamine, abgekürzt ABA, zum MDA.

Vor allem in die Umlagerungen greift ebenfalls US-A-5 310 769 ein. US-A-5 310 769 beschreibt einen Prozess zur Herstellung von Polyaminen der Diphenylmethanreihe durch Kondensation von Anilin mit Formaldehyd, nachfolgender Reaktion in Gegenwart eines sauren Katalysators, Neutralisation des sauren Katalysators nach vollständiger Reaktion und Reinigung des resultierenden Di- und Polyamingemischs durch Abdestillieren des überschüssigen aromatischen Amins dadurch gekennzeichnet, dass in einer bevorzugten Variante
a) Anilin mit Formaldehyd in einem Molverhältnis von 1,5:1 bis 10:1 bei Temperaturen zwischen 10 und 150°C umgesetzt wird,
b) danach dem Reaktionsgemisch ein saurer Katalysator im Molverhältnis Anilin zu saurem Katalysator von 2:1 bis 100:1 bei Temperaturen zwischen 10 und 150°C zugegeben wird, dabei das bei der Kondensationsreaktion entstandene Wasser entweder vor oder nach Schritt b) abgetrennt wird,
c) anschließend die Temperatur der in Schritt b) erhaltene Mischung um mindestens 40°C innerhalb von 15 Minuten erhöht und dann weiter auf die Endtemperatur zwischen 105 und 200°C aufgeheizt und für 10 bis 300 Minuten nach der Temperaturerhöhung gehalten wird.

US-A-5 310 769 lehrt, dass durch die besondere Temperaturführung während der Kondensations-und Umlagerungsschritte ein Di- und Polyamingemisch der Diphenylmethanreihe erhalten wird, dessen nachfolgende Phosgenierung besonders helle Polyurethanschäume zugänglich macht.

Di- und Polyamingemische der Diphenylmethanreihe, deren nachfolgende Phosgenierung zu Polyisocyanaten stark reduzierter Färbung führt, werden gemäß US-A-4 792 624 auch bei Anwendung eines Verfahrens erhalten, das dadurch gekennzeichnet ist, dass
a) schnell fließende Ströme von wässrigem Anilinhydrochlorid und wässrigem Formaldehyd in einem Verhältnis von 1.6 bis 8 Molen Anilin pro Mol Formaldehyd am Einlauf eines Rohrreaktors intensiv durchmischt werden, dadurch sofort eine Aminobenzylamine enthaltende Mischung erzeugt wird,
b) die gemäß a) erzeugte Mischung nachfolgend über eine gekühlte Reaktionszone geführt wird, in der der Gehalt an Aminobenzylaminen in der Mischung mindestens auf 30 Gew.% ansteigt,
c) das Reaktionsgemisch der gekühlten Reaktionszone in dem Maß entnommen wird, wie Reaktionsmischung aus Schritt a) nachfließt,
d) das Reaktionsgemisch der gekühlte Reaktionszone dann durch eine Umlagerungszone mit Temperaturen von 60° bis 200°C geführt wird, dabei das Polyamin der Diphenylmethanreihe gebildet wird,
e) das Reaktionsgemisch der Umlagerungszone in dem Maß entnommen wird, wie Reaktionsmischung in die Umlagerungszone gespeist wird,
f) das Reaktionsgemisch aus der Umlagerungszone kontinuierlich einer Neutralisationszone, in der die sauren Komponenten neutralisiert werden, zugeführt wird, dann Anilin und Wasser von dem Reaktionsgemisch abgetrennt werden, so ein Anilin-freies Polyamin der Diphenylmethanreihe erhalten wird,
g) das Polyamingemisch in dem Maß Schritt f) entnommen wird, wie Reaktionsmischung in die Neutralisations- bzw. Destillationsstufe eingespeist wird,
h) die Hauptmenge des gewonnenen Polyamingemischs in einen Lagertank ausgeschleust wird, ein Teilstrom des Polyamingemischs im Umfang von 1 bis 40 Gew.%, bezogen auf die vereinigten anfänglichen Gewichte der von in Schritt a) eingespeisten Anilin-, Anilinhydrochlorid- und Formaldehydmengen, jedoch auf Schritt b) zurückgeführt und bei laufendem Prozess a) bis h) erneut durch die Schritte b) bis h) geschleust wird.

Gemäß US-A-4 792 624 ist es zur Erzielung einer maximalen Farbwertverbesserung dabei wesentlich, dass das in den Prozess eingesetzte überschüssige Anilin von dem MDA abgetrennt wird, bevor das MDA den Benzylaminen zugesetzt wird. Darüber hinaus wird nach der Lehre von US-A-4 792 624 die verbesserte Färbung nur erreicht, wenn das zurückgeführte Polyamin dort zugesetzt wird, wo Aminobenzylamine vorhanden sind, und nicht in der Stufe, in der Anilin und Formaldehyd zuerst reagieren.

EP 1 813 598 A1 lehrt, dass sich der Einsatz von Anilin, das weniger als 3 Gew.%, bevorzugt 0,001 bis 3 Gew.%, besonders bevorzugt 0,01 bis 1 Gew.% Di- und Polyamine der Diphenylmethanreihe, bezogen auf das Gewicht des eingesetzten Anilins, enthält, bei der Herstellung von MDA vorteilhaft auf die Farbe des daraus durch Phosgenierung hergestellten MDI auswirkt. Die im Anilin enthaltenen Di- und Polyamine der Diphenylmethanreihe gelangen in das zur Herstellung von MDA eingesetzte Amin, da üblicherweise bei der MDA-Herstellung Anilin im Überschuss eingesetzt wird, und der Überschuss nach z.B. destillativer Abtrennung vom Produkt wieder in den Prozess zurückgeführt wird.

Weitere im Anilin enthaltene Nebenkomponenten sind z.B. in RD 510004 (Research Disclosure Journal, Veröffentlichung Oktober 2006) aufgeführt, so z.B. Cyclohexylamin, Cyclohexanol, Cyclohexanon, Phenol usw., ohne dass erwähnt wird, in welchen Gehalten diese Nebenkomponenten im Anilin enthalten sind und ob und welche Einflüsse diese Nebenkomponenten auf die Qualität des MDA's und des daraus gewonnenen MDI's haben.

Vielen der vorstehend zitierten und in der Literatur beschriebenen Verfahren zur Herstellung von Di- und Polyaminen der Diphenylmethanreihe durch Umsetzung von Anilin mit Formalin in Gegenwart saurer Katalysatoren ist gemeinsam, dass sie durch Modifikation einzelner Verfahrensparameter, z.B. der Komponentendosierung, der Konzentration an saurem Katalysator, der Temperaturführung oder auch der Produktzusammensetzung bei den Umlagerungen, jeweils Verbesserungen bei der Färbung der erzeugten Di- und Polyamingemische der Diphenylmethanreihe bzw. der aus ihnen erzeugten Isocyanate und Polyurethane erreichen. Dennoch besteht weiterhin ein Bedarf an neuen Verfahren mit noch weitergehenden Farbverbesserungen.

Diese Aufgabe konnte überraschenderweise dadurch gelöst werden, dass bei der Herstellung der Di- und Polyamine der Diphenylmethanreihe durch Umsetzung von Anilin und Formaldehyd in Gegenwart saurer Katalysatoren ein Anilin eingesetzt wird, das in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen. Die Erfindung betrifft ein Verfahren zur Herstellung von Di- und Polyaminen der Diphenylmethanreihe durch Umsetzung von Anilin und Formaldehyd in Gegenwart eines sauren Katalysators, bei dem das eingesetzte Anilin in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten und die (cyclo)aliphatische Ketone, welche ggf. ungesättigt und/oder substituiert sind, umfassen, oder die durch Reaktion dieser wenigstens eine Carbonylgruppe enthaltenden Verbindungen mit Anilin entstehen können und die Schiff'sche Basen umfassen.

Die vorstehend gemachten Angaben verstehen sich als Summe der Gehalte der einzelnen Carbonylgruppen enthaltenden Verbindungen und der daraus durch Reaktion mit Anilin entstandenen Verbindungen. Bevorzugt können einzelne Carbonylgruppen enthaltende Verbindungen oder einzelne der daraus durch Reaktion mit Anilin entstehenden Verbindungen einen Gehalt von weniger als 0,4 Gew.%, bevorzugt 0,0001 bis 0,3 Gew.%, besonders bevorzugt 0,0001 bis 0,25 Gew.%, und insbesondere bevorzugt 0,0001 bis 0,2 Gew.%, bezogen auf das Gewicht des eingesetzten Anilins, haben.
Die Verbindungen, die wenigstens eine Carbonylgruppen enthalten, und die Verbindungen, die durch Reaktion dieser wenigstens eine Carbonylgruppe enthaltenden Verbindungen mit Anilin entstehen, können entweder dem frisch eingesetzten Anilin entstammen, sind im zurückgeführten Anilin enthalten oder werden im Laufe der Herstellung von Di- und Polyaminen der Diphenylmethanreihe als Nebenprodukte gebildet. Sie können ggf. im Anilinkreislauf angereichert werden und Gehalte erreichen, die über denjenigen im frischen Anilin liegen.
Neben den wenigstens eine Carbonylgruppen enthaltenden Verbindungen und den Verbindungen, die durch Reaktion dieser wenigstens eine Carbonylgruppe enthaltenden Verbindungen mit Anilin entstehen können, sind im Anilin im Allgemeinen auch noch weitere Verunreinigungen enthalten, die die Farbe des MDI negativ beeinflussen können. Allerdings ist deren Einfluss auf die Farbe des MDI nach Phosgenierung des MDA nicht so ausgeprägt. Dennoch sollte der Gehalt dieser Verunreinigungen im Anilin möglichst gering gehalten werden. Diese im Anilin im Allgemeinen enthaltenen Verunreinigungen umfassen u.a. die folgenden Verbindungen bzw. Verbindungsklassen, wobei die Auflistung als beispielhaft und nicht limitierend anzusehen ist: ggf. ungesättigte und/oder substituierte (cyclo)aliphatische Kohlenwasserstoffe wie Cyclohexan, Cyclohexen und Methylcyclohexan, ggf. substituierte aromatische Kohlenwasserstoffe wie Benzol, Toluol, Ethylbenzol und die isomeren Xylole, Nitroaromaten wie Nitrobenzol, die isomeren Dinitrobenzole, die isomeren Nitrotoluole und die isomeren Dinitrotoluole, ggf. ungesättigte und/oder substituierte (cyclo)aliphatische Alkohole wie Cyclohexanol, ggf. substituierte Phenole wie Phenol und die isomeren Kresole, ggf. ungesättigte und/oder substituierte (cyclo)aliphatische primäre, sekundäre oder tertiäre Amine wie Cyclohexylamin, N,N-Dicyclohexylamin und N-Methylcyclohexylamin, ggf. am Aromaten oder am Stickstoff substituierte aromatische primäre, sekundäre oder tertiäre Mono-, Di- und Polyamine wie o-Toluidin, m-Toluidin, p-Toluidin, o-Phenylendiamin, m-Phenylendiamin, p-Phenylendiamin, N-Methylanilin, N-Ethylanilin, N,N-Diethylanilin, die isomeren N-Ethyltoluidine, die isomeren N,N-Diethyltoluidine, N-Cyclohexylanilin, Diphenylamin, o-Aminophenol, m-Aminophenol, p-Aminophenol und o-Aminodiphenyl, Di- und Polyamine der Diphenylmethanreihe (MDA), Stickstoff enthaltende Heteroaromaten oder davon abgeleitete Strukturen wie Phenazin, Acridin, Acridan, Pyridin und ggf. substituierte und/oder ggf. (teil)gesättigte Chinazoline wie N-Phenyldihydrochinazolin und N-Phenyltetrahydrochinazolin.

Während die Aminogruppen enthaltenden Verunreinigungen im Anilin bereits im Stand der Technik bekannt sind und in vielen Fällen als mögliche Ursache für Qualitätsprobleme (z.B. für erhöhte Farbwerte in den erzeugten Di- und Polyamingemischen der Diphenylmethanreihe bzw. der aus ihnen erzeugten Isocyanate und Polyurethane) identifiziert wurden, sind einige im Anilin enthaltene Verbindungen/Verbindungsklassen als weitgehend inert anzusehen. Sie kommen als Ursachen für merkliche Qualitätsprobleme, u.a. erhöhte Farbwerte, in den erzeugten Di- und Polyamingemischen der Diphenylmethanreihe bzw. den aus ihnen erzeugten Isocyanaten und Polyurethanen kaum in Frage.

Dies gilt z.B. für ggf. ungesättigte und/oder substituierte (cyclo)aliphatische Alkohole wie Cyclohexanol, dessen Anwesenheit im Anilin nicht zu erhöhten Farbwerten in den erzeugten Di-und Polyamingemischen der Diphenylmethanreihe bzw. den aus ihnen erzeugten Isocyanaten und Polyurethanen führt.

Daher ist es völlig überraschend, dass die zu den weitgehend inerten Alkoholen strukturell ähnliche Verbindungsklasse der Carbonylgruppen enthaltenden Verbindungen (insbesondere die Verbindungsklasse der ggf. ungesättigten und/oder substituierten (cyclo)aliphatischen Ketone wie z.B. Cyclohexanon, und die daraus durch Reaktion mit Anilin entstandenen Produkte, z.B. Schiff'sche Basen wie N-Phenylcyclohexylimin) merkliche Qualitätsprobleme und insbesondere erhöhte Farbwerte in den erzeugten Di- und Polyamingemischen der Diphenylmethanreihe bzw. den aus ihnen erzeugten Isocyanaten (Di- und Polyisocyanate der Diphenylmethanreihe) und Polyurethanen verursachen.

Der erfindungsgemäße Einsatz von Anilin enthaltend in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen, die wenigstens eine Carbonylgruppe enthalten oder die durch Reaktion dieser wenigstens eine Carbonylgruppe enthaltenden Verbindungen mit Anilin entstehen, ist insofern von besonderer Bedeutung, dass die Umsetzung von Anilin mit Formaldehyd in Gegenwart saurer Katalysatoren zur Einstellung des gewünschten Gehalts an Diaminen sowie zur Wahrung der Handhabbarkeit der Reaktionsgemische stets mit einem Anilinüberschuss durchgeführt wird. Dieser Anilinüberschuss muss bei der Aufbereitung der Di- und Polyamingemische der Diphenylmethanreihe abgetrennt und zur Wahrung der äußeren Massenbilanz wieder auf die Reaktionsstufen des Verfahrens zurückgeführt werden. Im Zuge der MDA-Synthese verbrauchtes Anilin wird in Form von frischem Anilin ergänzt. Bevorzugt wird also das erfindungsgemäße Verfahren zur Herstellung von Di- und Polyaminen der Diphenylmethanreihe durch Umsetzung von Anilin und Formaldehyd in Gegenwart eines sauren Katalysators, bevorzugt von Salzsäure, so durchgeführt, dass das Anilin im stöchiometrischen Überschuss eingesetzt wird, das überschüssige Anilin nach der Reaktion abgetrennt wird und zumindest teilweise wieder in die Reaktion von Anilin und Formaldehyd zurückgeführt wird.

Die Abtrennung des Anilinüberschusses aus dem bei der Umsetzung von Anilin und Formaldehyd erhaltenen Reaktionsgemisch geschieht üblicherweise destillativ, wobei ebenfalls das dem Polyamingemisch noch anhaftende Wasser abgetrennt wird.

Ggf. können zur Reinigung der Abwässer, die bei der Umsetzung von Anilin mit Formalin (Formaldehyd) in Gegenwart saurer Katalysatoren mit nachfolgender Neutralisation und Separation und Aufbereitung der organischen Phase anfallen, die Abwässer - wie gemäß dem Stand der Technik üblich (JP 2004026753) - mit einem zusätzlich eingesetzten hydrophoben Lösungsmittel extrahiert werden. Alternativ können zur Reinigung der Abwässer die Abwässer mit den nicht weiter aufbereiteten Kondensaten aus der Anilinabtrennung oder frischem Anilin vermischt werden, woran sich eine Phasentrennung anschließt. Die dabei erhaltene organische Phase kann dann wie oben beschrieben in die Synthesestufen des Verfahrens zurückgeführt werden.

Die Erfindung betrifft bevorzugt ein Verfahren zur Herstellung von Di- und Polyaminen der Diphenylmethanreihe, bei dem
a) Anilin und Formaldehyd in Gegenwart eines sauren Katalysators zu einem Reaktionsgemisch enthaltend Di- und Polyamine umgesetzt werden, wobei das eingesetzte Anilin in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen, und
b) das Reaktionsgemisch enthaltend Di- und Polyamine neutralisiert wird, und
c) das neutralisierte Reaktionsgemisch enthaltend Di- und Polyamine in eine organische Phase enthaltend Di- und Polyamine und eine wässrige Phase aufgetrennt wird, und
d) ggf. die organische Phase mit Wasser gewaschen wird, und
e) aus der organischen Phase überschüssiges Anilin destillativ entfernt wird, und
f) die in den Schritten a) - e) anfallenden Abwässer und Kondensate teilweise oder vollständig zusammengeführt werden, wobei zumindest die in den Schritten c) und e) erhaltenen Abwässer und Kondensate jeweils zumindest teilweise zusammengeführt werden, und wobei eine Mischung enthaltend Wasser, Di- und Polyamine, Anilin und Salze des in Schritt a) eingesetzten Katalysators erhalten wird, und
g) die in Schritt f) erhaltene Mischung einer Phasentrennung unterzogen wird, wobei ein Anilin enthaltend Wasser sowie Di- und Polyamine erhalten wird, und
h) das bei der Phasentrennung erhaltene Anilin anschließend zumindest teilweise in die Umsetzung in Schritt a) zurückgeführt wird.

Bevorzugt wird das bei der Phasentrennung in Schritt g) erhaltene Anilin enthaltend Wasser sowie Di- und Polyamine zusätzlich teilweise in einen der Schritte b) bis e) eingespeist.

Bevorzugt wird das in Schritt g) gewonnene extrahierte Abwasser einer Extraktion mit Anilin, bevorzugt frisch eingesetztem Anilin, unterzogen. Weiterhin bevorzugt werden die dabei erhaltenen Extrakte bevorzugt der in Schritt f) erhaltenen Mischung zugesetzt.

Der zur Erzeugung von Di- und Polyamingemischen mit niedrigen Farbwerten beschriebene Einsatz von Anilin, das in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen, kann in allen bekannten Verfahren zur Herstellung von MDA aus Anilin und Formaldehyd in Gegenwart saurer Katalysatoren erfolgen. Bevorzugt wird jedoch als saurer Katalysator Salzsäure eingesetzt. Wesentlich ist nur, dass die angegebenen Grenzen des Gehalts der vorstehend genannten Verunreinigungen im eingesetzten Anilin in den Apparaten der ein- oder mehrstufigen Umsetzung des Anilins mit Formaldehyd eingehalten werden und örtliche Überkonzentrationen vermieden werden. Bevorzugt wird jedoch als saurer Katalysator ein homogener Katalysator, besonders bevorzugt Salzsäure, eingesetzt. Beim Einsatz heterogener Katalysatoren sind die ablaufenden Reaktionsmechanismen durch andere Einflüsse, wie beispielsweise Transportphänomene, überlagert.

Bevorzugt wird das in Schritt a) eingesetzte Anilin durch Vereinigung zumindest einer Teilmenge des in Schritt g) erhaltenen Anilins enthaltend Di- und Polyamine mit aus anderen Quellen stammendem Anilin, bevorzugt frischem Anilin, hergestellt. Dabei ist es wesentlich, dass das eingesetzte Anilin in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen. Etwaige Überschüsse des in Schritt g) erhaltenen Anilins enthaltend Wasser sowie Di- und Polyamine werden bevorzugt der Neutralisation und / oder Aufbereitung der Di- und Polyamingemische (Schritte b) - e)) zugeführt.

Diese Verfahrensweise stellt zum einen den für die Herstellung von hellen Di- und Polyamingemischen der Diphenylmethanreihe bzw. den aus ihnen hergestellten Isocyanaten und Polyurethanen notwendigen niedrigen Gehalt an Di- und Polyaminen in dem in der Reaktion in Schritt a) eingesetzten Anilin sicher. Zum anderen minimiert diese Verfahrensweise vorteilhaft den zur Aufbereitung der Polyamingemische in Schritt e) notwendigen Aufwand sowie den zur Aufbereitung der in Schritt g) erhaltenen Extrakte notwendigen Energieeinsatz. Denn die Herstellung der Di und Polyamine der Diphenylmethanreihe erfolgt industriell in einem so großen Maßstab, dass selbst geringe ökonomische Verbesserungen solch wichtiger großtechnischer Verfahren von großem wirtschaftlichem Interesse sind.

Bevorzugt wird in Schritt a) das Anilin, das Anilin in der Summe weniger als 0,5 Gew.%, bevorzugt 0,0001 bis 0,4 Gew.%, besonders bevorzugt 0,0001 bis 0,3 Gew.%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen, zunächst mit Formaldehyd in einem Molverhältnis von bevorzugt 1,6:1 bis 10:1, besonders bevorzugt zwischen 1,6: 1 und 4,0:1, und bei einer Temperatur von bevorzugt zwischen 10 und 150°C, besonders bevorzugt zwischen 75 und 110°C, umgesetzt.

Dann wird dem Reaktionsgemisch bevorzugt ein saurer Katalysator in einem Molverhältnis Anilin zum sauren Katalysator von bevorzugt 2:1 bis 100:1 (entsprechend einem Protonierungsgrad des Anilins von 50 bis 1 %), besonders bevorzugt 4:1 bis 20:1, bei einer Temperatur von bevorzugt 10 bis 150 °C, besonders bevorzugt 35 bis 75°C zugegeben. Als saurer Katalysator wird bevorzugt Salzsäure eingesetzt.

Das bei der Kondensation des Formaldehyds mit dem Anilin entstandene und ggf. mit dem Formaldehyd eingetragene Wasser wird dabei bevorzugt ganz oder teilweise vor der Katalysatorzugabe (z.B. durch Phasentrennung) oder danach (z.B. durch Siedekühlung und Austrag der dabei gewonnenen Kondensate) abgetrennt.

Das Reaktionsgemisch wird anschließend bevorzugt auf Temperaturen von zwischen 100 bis 180°C, besonders bevorzugt von zwischen 130 - 160°C, aufgeheizt und nach Erreichen der Endtemperatur bei dieser Temperatur für 5 bis 300 Minuten gehalten.

In Schritt b) wird das Reaktionsgemisch enthaltend MDA anschließend ggf. unter Zusatz von Wasser und / oder Anilin neutralisiert. Bevorzugt erfolgt die Neutralisation mit Natronlauge.

Anschließend wird in Schritt c) das neutralisierte Reaktionsgemisch enthaltend MDA in eine organische Phase enthaltend MDA und eine wässrige Phase aufgetrennt. Dies kann durch die Zugabe von Anilin und/oder Wasser unterstützt werden. Wird die Phasentrennung durch Zugabe von Anilin und/oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und/oder Wasser verdünnte Reaktionsgemisch wird anschließend bevorzugt einem Apparat zugeführt, der aufgrund seiner Konfiguration und/oder Einbauten besonders zur Auftrennung in eine organische Phase enthaltend MDA und eine wässrige Phase geeignet ist. Zum Einsatz kommen bevorzugt Scheideflaschen mit die Koaleszenz der beiden Phasen unterstützenden Plattenpaketen als Einbauten.

In Schritt d) wird ggf. die organische Phase enthaltend MDA bevorzugt bei Temperaturen von zwischen 50 und 150 °C, besonders bevorzugt von zwischen 80 und 110 °C mit Wasser in einem Verhältnis von Wasser zu organischer Phase von 0,05- 2 : 1 gewaschen.

Die in Schritt c) erhaltene organische Phase wird, ggf. nach erfolgter Wäsche in Schritt d), anschließend in Schritt e) in einer Destillation von Anilin gereinigt, wobei ein gereinigtes MDA und ein Kondensat enthaltend Anilin und Wasser erhalten wird.

Die in den Schritten a) bis e) erhaltenen Abwässer, wie die wässrige Phase aus Schritt c), das Waschwasser aus Schritt d) und das Kondensat aus Schritt e) und ggf. weitere Prozesswässer wie beispielsweise weitere kondensierte Brüden oder in Schritt a) erhaltene wässrige Phasen, werden anschließend teilweise oder vollständig in Schritt f) vereinigt. Dabei werden zumindest die in den Schritten c) und e) erhaltenen Abwässer und Kondensate jeweils zumindest teilweise, bevorzugt jeweils zu wenigstens 50%, zusammengeführt. Dabei wird eine Mischung erhalten, welche bevorzugt Wasser sowie 0,001- 5 Gew.% MDA, 0,5- 60 Gew.% Anilin und 1- 25 Gew.% an Salzen des in Schritt a) eingesetzten sauren Katalysators, jeweils bezogen auf das Gewicht der Mischung, enthält.

Die in Schritt f) erhaltene Mischung wird dann in Schritt g) bevorzugt bei einer Temperatur von zwischen 30 und 120 °C, bevorzugt 70 - 110 °C , einer Phasentrennung unterworfen, wobei ein Anilin enthaltend Di- und Polyamine erhalten wird.

Fakultativ kann das in Schritt g) erhaltenen Abwasser in einem weiteren Schritt einer Extraktion mit Anilin, bevorzugt mit frisch eingesetztem Anilin, bevorzugt bei einer Temperatur von zwischen 30 und 120 °C, bevorzugt 70 - 110 °C, mit Anilin im Gewichts-Verhältnis von Anilin zu Abwasser von bevorzugt 0,05 -1: 1, bevorzugt 0,1 - 0,3:1, extrahiert werden, wobei die Extrakte vorteilhaft der in Schritt f) bereiteten Mischung zugeführt werden. Dabei erfolgt die Extraktion bevorzugt mehrstufig und im Gegenstrom. Bevorzugt wird Anilin als alleiniges Extraktionsmittel eingesetzt.

Vorteilhaft unterstützt wird die Extraktion mit frisch eingesetztem Anilin durch die in Schritt f) durchgeführte Phasentrennung (die mit einer Extraktion einhergeht) und die in Schritt g) durchgeführte Extraktion der Abwässer mit den Kondensaten der in Schritt e) durchgeführten destillativen Entanilinierung. Es sind damit sehr geringe Einsätze an Frischanilin zur Extraktion erforderlich. Dabei werden aufgrund der Phenolfreiheit der Kondensate geringere Phenolbeladungen im extrahierten Abwasser erhalten.

Vorteilhaft ist die destillative Entfernung des Anilins aus dem extrahierten Abwasser. Anilin bildet mit Wasser ein niedrig siedendes Azeotrop, so dass die destillative Entfernung des Anilins bei einem (ggf. nur leicht) vermindertem Druck, der sich einfach einstellen und technisch handhaben lässt, bei Temperaturen unter 100 °C durchgeführt werden kann, dann sogar Abwärmen vorteilhaft als Energien bei der destillativen Rückgewinnung des Anilins eingesetzt werden können.

Die bei der destillativen Anilinentfernung aus dem extrahierten Abwasser erhaltenen Kondensate enthalten einen hohen Wasseranteil. Die Kondensate können deshalb vorteilhaft ganz oder teilweise als Verdünnungswasser in der Neutralisation in Schritt b) und als Waschwasser in Schritt d) eingesetzt werden, besonders bevorzugt erst als Waschwasser in Schritt d), dann als Verdünnungswasser in der Neutralisation in Schritt b). Dabei wird ein großer Waschmitteleinsatz möglich, ohne das Abwasseraufkommen des Verfahrens zu vergrößern.

Werden bei der destillativen Anilinentfernung aus dem extrahierten Abwasser die Brüden mehrstufig kondensiert, kann vorteilhaft eine Fraktion enthaltend Methanol und weitere Leichtsieder in hohen Konzentrationen erzeugt werden. Zum einen wird damit der Methanolpegel in den Verfahrensstufen vorteilhaft abgesenkt, zum anderen kann diese Fraktion vorteilhaft als Brennstoffsubstitut eingesetzt werden.

In Schritt h) wird schließlich das bei der Phasentrennung in Schritt g) erhaltene Anilin enthaltend Wasser sowie Di- und Polyamine teilweise oder vollständig in Schritt a) zurückgeführt. Bevorzugt wird dabei das zurückgeführte Anilin ohne weitere Aufarbeitung in der Reaktion in Schritt a) eingesetzt. Ggf. wird ein weiterer Teil in einen der Prozessschritte b) - e) eingesetzt.

Das erfindungsgemäße Verfahren zeichnet sich zum einen durch eine besonders einfache Handhabung der bei der extraktiven Aufarbeitung seiner Abwässer und Kondensate anfallenden Anilin-haltigen Ströme aus. Die nach dem erfindungsgemäßen Verfahren hergestellten Polyamine weisen darüber hinaus geringe Gehalte an Chromophoren auf und können vorteilhaft zu gering gefärbten Isocyanaten bzw. hellen Polyurethanprodukten umgesetzt werden. Dabei kann die Phosgenierung der Polyamine sowie die Isolierung der dabei erhaltenen Polyisocyanate und deren Umsetzung zu Polyurethanprodukten gemäß den bekannten technischen Verfahren durchgeführt werden.

Neu und wesentlich an dem erfindungsgemäßen Verfahren ist, dass in der Reaktion von Anilin und Formaldehyd ein Anilin eingesetzt wird, das in der Summe weniger als 0,5 Gew.%, bevorzugt 0,0001 bis 0,4 Gew.%, besonders bevorzugt 0,0001 bis 0,3 Ges.-%, und insbesondere bevorzugt 0,0001 bis 0,25 Gew.%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen. Durch den geringen Gehalt an Carbonylgruppen enthaltenden Verunreinigungen und an Verbindungen, die durch Reaktion dieser wenigstens eine Carbonylgruppe enthaltenden Verbindungen mit Anilin entstehen in dem eingesetzten Anilin werden verbesserte Farbwerte der Di- und Polyamine der Diphenylmethanreihe und der daraus hergestellten Di- und Polyisocyanate der Diphenylmethanreihe bzw. Polyurethane erhalten.

Bevorzugt wird das erfindungsgemäße Verfahren so durchgeführt, dass die Extraktion mit Anilin nach der Phasentrennung des neutralisierten Reaktionsgemisches enthaltend MDA erfolgt, dabei nur die Extraktion der wässrigen Phase mit Anilin erfolgt und dazu vorwiegend die in Schritt e) erhaltenen Anilin-haltigen Kondensate der destillativen Anilinentfernung als Extraktionsmittel in Schritt g) eingesetzt werden. Denn die Phasentrennung in Schritt g) erfolgt faktisch unter gleichzeitiger Extraktion der wässrigen Phase durch das in den stark Anilin-haltigen Kondensaten enthaltene Anilin (organische Phase). Das noch beide Phasen enthaltende Reaktionsgemisch oder die nach der Phasentrennung erhaltene organische Phase wird nach dem erfindungsgemäßen Verfahren nicht mit Anilin extrahiert. Durch die Abtrennung der Abwässer und den Einsatz der Kondensate können im Unterschied zum Stand der Technik der Einsatz und die Aufarbeitung eines zusätzlichen hydrophoben Lösungsmittels entfallen und sind - wenn überhaupt - nur äußerst geringe Mengen an frisch eingesetztem Anilin zur Aufbereitung der Abwässer erforderlich.

Die Färbung der Di- und Polyisocyanate kann durch zwei Absorptionsmaxima im sichtbaren UV-Bereich bei 430 bzw. 520 nm charakterisiert werden. Dabei kann aufgrund entsprechender Erfahrung aus diesen Werten die Färbung der aus den Di- und Polyisocyanaten der Diphenylmethanreihe hergestellten Polyurethanprodukte vorhergesagt werden. Der Wert bei 430 nm entspricht einer gelb-braunen, der Wert bei 520 nm einer grauen Färbung. Kleineren Absorptionswerten der Di- und Polyisocyanate entsprechen geringere bzw. hellere Färbungen der aus diesen Di- und Polyisocyanaten der Diphenylmethanreihe hergestellten Polyurethanprodukte.

Die erfindungsgemäßen Verfahren sollen anhand der folgenden Beispiele näher dargestellt werden:

### Beispiele

### Allgemeine Arbeitsvorschrift zur Herstellung von MDA:

### Ausgangsmaterialien:

- Anilin
- Formaldehyd, 32 Gew.%ige Lösung in Wasser (auch Formalin genannt)
- 30 Gew.%ige wässrige Salzsäure
- 50 Gew.%ige Natronlauge
- destilliertes Wasser

Zu 279 g reinem Anilin (d.h. Anilin, das keine messbaren Gehalte an Verbindungen enthält, die wenigstens eine Carbonylgruppe enthalten oder die durch Reaktion dieser wenigstens eine Carbonylgruppe enthaltenden Verbindungen mit Anilin entstehen) dem ggf. Cyclohexanol, Cyclohexanon bzw. N-Phenylcyclohexylimin beigesetzt wird (siehe Tabelle 1), werden bei 80°C unter Rühren innerhalb von 20 min 134 g einer 32 Gew.%igen wässrigen Formaldehyd-Lösung getropft. Nach der Zugabe wird noch 5 min gerührt und bei 80°C eine Phasentrennung vorgenommen. Die organische Phase, das Aminal, wird anschließend bei 35°C innerhalb von 30 min unter Rühren mit 91 g einer 30 Gew.%igen wässrigen Salzsäure versetzt. Nach 30 min Rühren bei 35°C wird das Reaktionsgemisch auf 60°C aufgeheizt und weitere 30 min bei 60°C gerührt. Anschließend wird die Reaktionstemperatur auf 104°C erhöht. Bei dieser Temperatur wird das Reaktionsgemisch für weitere 10 Stunden gerührt, um die Umsetzung zu vervollständigen.

Das so hergestellte saure Reaktionsgemisch wird in einem Rührgefäß bei einer Temperatur von 95-100°C unter Rühren mit 72 g einer 50 Gew.%igen Natronlauge und 100 g Wasser versetzt. Das entstandene zweiphasige Gemisch wird ca. 15 min bei 95-100°C nachgerührt. Nach Abtrennung der wässrigen Phase wird die organische Phase zweimal mit jeweils 300 ml siedendem, destilliertem Wasser gewaschen, indem nach Zugabe des Wassers unter Rühren für ca. 5 min auf Rückflusstemperatur erhitzt und anschließend die wässrige Phase abgetrennt wird.

Nach der zweiten Wäsche wird die organische Phase in eine Destillationsapparatur überführt. Im Wasserstrahlvakuum (ca. 20 mbar) werden Wasser und Anilin abdestilliert. Die Destillation wird abgebrochen, wenn die Sumpftemperatur ca. 280°C erreicht und 2-Kern-MDA zu destillieren beginnt. Der Destillationssumpf (Produkt) wird abgekühlt, nach Belüften der Destillationsapparatur mit Stickstoff in ein Vorratsgefäß überführt und unter Stickstoff gelagert. Das erhaltene MDA hat einen 2-Kern-Anteil von ca. 63 %.

### Allgemeine Arbeitsvorschrift zur Phosgenierung:

### Ausgangsmaterialien:

- MDA, erhalten durch sauer katalysierte Kondensation von Anilin mit Formaldehyd gemäß der vorstehenden allgemeinen Arbeitsvorschrift
- Chlorbenzol wasserfrei
- Phosgen

50 g des MDA's werden in 255 ml Chlorbenzol gelöst, auf 55°C erwärmt und innerhalb von 10 s unter intensivem Rühren zu einer auf 0°C temperierten Lösung von 105 g Phosgen in 310 ml Chlorbenzol gegeben. Die Suspension wird unter Durchleiten von Phosgen innerhalb von 45 min auf 100°C und anschließend während 10 Min auf Rückflusstemperatur aufgeheizt. Nach weiteren 10 min bei dieser Temperatur wird das Lösungsmittel unter vermindertem Druck bis zu einer Sumpftemperatur von 100°C abdestilliert. Das rohe Isocyanat wird anschließend in einer Destillationsapparatur bei einem Druck von 4 bis 6 mbar mittels eines Heißluftgebläses bis zum ersten Produktübergang erhitzt und daraufhin innerhalb von 5 bis 10 min auf Raumtemperatur abgekühlt. Von dem so erhaltenen Isocyanat wird 1,0 g in Chlorbenzol gelöst und mit Chlorbenzol zu 50 ml verdünnt. Die Extinktion der so erhaltenen Lösung wird bei den beiden Wellenlängen 430 nm und 520 nm bestimmt. Als Messgerät wird ein Photometer Dr. Lange LICO 300 eingesetzt. Die Ergebnisse sind in Tabelle 1 zusammengefasst.

**Tabelle 1**

| | Zusatz | | E 430 | E 520 |
|---|---|---|---|---|
| | | [Gew.-%] | | |
| Standard | - | - | 0,154 | 0,024 |
| Vergleichsbeispiel | Cyclohexanol | 0,5 | 0,134 | 0,021 |
| Beispiel 1 | Cyclohexanon | 0,05 | 0,189 | 0,034 |
| Beispiel 2 | Cyclohexanon | 0,1 | 0,221 | 0,042 |
| Beispiel 3 | Cyclohexanon | 0,5 | 1,016 | 0,047 |
| Beispiel 4 | Cyclohexanon | 1,0 | 1,676 | 0,074 |
| Beispiel 5 | Cyclohexanon | 2,0 | 2,624 | 0,135 |
| Beispiel 6 | N-Phenylcyclohexylimin | 0,05 | 0,172 | 0,028 |
| Beispiel 7 | N-Phenylcyclohexylimin | 0,5 | 0,629 | 0,026 |
| Beispiel 8 | N-Phenylcyclohexylimin | 1,0 | 1,106 | 0,042 |
| Beispiel 9 | N-Phenylcyclohexylimin | 2,0 | 1,947 | 0,083 |

### Ergebnis:

Der Vergleich des Standards und der erfindungsgemäßen Beispiele 1, 2 und 6 mit den übrigen, nicht erfindungsgemäßen Beispielen verdeutlicht, dass Cyclohexanon und das daraus durch Reaktion mit Anilin sich bildende N-Phenylcyclohexylimin in höheren Konzentrationen stark farbverursachende Verbindungen sind. Dies zeigt sich an den stark erhöhten Extinktionswerten bei 430 und 520nm. Gleichzeitig zeigt der Vergleich des Vergleichsbeispiels mit dem entsprechenden Standard, dass, obwohl Cyclohexanol strukturell sehr ähnlich zu Cyclohexanon ist, Cyclohexanol dennoch kaum farb-verschlechternd wirkt. Daher ist die Erkenntnis, dass Verbindungen, die wenigstens eine Carbonylgruppe enthalten oder die durch Reaktion dieser wenigstens eine Carbonylgruppe enthaltenden Verbindungen mit Anilin entstehen, wie Cyclohexanon und das sich daraus durch Reaktion mit Anilin ergebende N-Phenylcyclohexylimin einen ausgeprägten negativen Effekt auf die Produktfarbe bewirken, als ausgesprochen überraschend einzustufen.

## Patentansprüche

1. Verfahren zur Herstellung von Di- und Polyaminen der Diphenylmethanreihe durch Umsetzung von Anilin und Formaldehyd in Gegenwart eines sauren Katalysators, bei dem das eingesetzte Anilin in der Summe weniger als 0,5 Gew.-%, bezogen auf das Gewicht des eingesetzten Anilins, an Verbindungen enthält,
die wenigstens eine Carbonylgruppe enthalten und die (cyclo)aliphatische Ketone, welche ggf. ungesättigt und/oder substituiert sind, umfassen, oder
die durch Reaktion dieser wenigstens eine Carbonylgruppe enthaltenden Verbindungen mit Anilin entstehen können und die Schiff'sche Basen umfassen.

2. Verfahren nach Anspruch 1, bei dem das eingesetzte Anilin in der Summe 0,0001 bis 0,4 Gew.-%, bezogen auf das Gewicht des eingesetzten Anilins, an Verbindungen enthält, die wenigstens eine Carbonylgruppe enthalten oder die durch Reaktion dieser wenigstens eine Carbonylgruppe enthaltenden Verbindungen mit Anilin entstehen.

3. Verfahren nach Anspruch 1, bei dem das eingesetzte Anilin in der Summe weniger als 0,4 Gew.-%, bezogen auf das Gewicht des eingesetzten Anilins, an ggf. ungesättigten und/oder substituierten (cyclo)aliphatischen Ketonen enthält.

4. Verfahren nach Anspruch 3, bei dem die Carbonylgruppen enthaltende Verbindung Cyclohexanon ist.

5. Verfahren nach Anspruch 1, bei dem das eingesetzte Anilin in der Summe weniger als 0,4 Gew.-%, bezogen auf das Gewicht des eingesetzten Anilins, an Schiff'schen Basen enthält.

6. Verfahren nach Anspruch 5, bei dem die Schiff'sche Base N-Phenylcyclohexylimin ist.

7. Verfahren zur Herstellung von Di- und Polyisocyanaten der Diphenylmethanreihe, bei dem Di- und Polyaminen der Diphenylmethanreihe nach dem Verfahren nach einem der Ansprüche 1 bis 6 hergestellt werden, und anschließend mit Phosgen zu den Di- und Polyisocyanaten der Diphenylmethanreihe umgesetzt werden.

## Claims

1. Process for producing diamines and polyamines of the diphenylmethane series by reacting aniline and formaldehyde in the presence of an acid catalyst, wherein the aniline used contains in total less than 0.5 wt.%, relative to the weight of aniline used, of compounds which contain at least one carbonyl group and which comprise (cyclo)aliphatic ketones which are optionally unsaturated and/or substituted or which can be formed by reaction of these compounds containing at least one carbonyl group with aniline and which comprise Schiff bases.

2. Process according to claim 1, wherein the aniline used contains in total 0.0001 to 0.4 wt.%, relative to the weight of aniline used, of compounds which contain at least one carbonyl group or which are formed by reaction of these compounds containing at least one carbonyl group with aniline.

3. Process according to claim 1, wherein the aniline used contains in total less than 0.4 wt.%, relative to the weight of aniline used, of optionally unsaturated and/or substituted (cyclo)aliphatic ketones.

4. Process according to claim 3, wherein the compound containing carbonyl groups is cyclohexanone.

5. Process according to claim 1, wherein the aniline used contains in total less than 0.4 wt.%, relative to the weight of aniline used, of Schiff bases.

6. Process according to claim 5, wherein the Schiff base is N-phenylcyclohexylimine.

7. Process for producing diisocyanates and polyisocyanates of the diphenylmethane series, wherein diamines and polyamines of the diphenylmethane series are produced by the process according to one of claims 1 to 6 and then reacted with phosgene to form the diisocyanates and polyisocyanates of the diphenylmethane series.

## Revendications

1. Procédé de fabrication de di- et polyamines de la série du diphénylméthane par mise en réaction d'aniline et de formaldéhyde en présence d'un catalyseur acide, selon lequel l'aniline utilisée contient au total moins de 0,5 % en poids, par rapport au poids de l'aniline utilisée, de composés
qui contiennent au moins un groupe carbonyle et qui comprennent des cétones (cyclo)aliphatiques, qui sont éventuellement insaturées et/ou substituées, ou
qui peuvent être formés par réaction de ces composés contenant au moins un groupe carbonyle avec de l'aniline et qui comprennent des bases de Schiff.

2. Procédé selon la revendication 1, selon lequel l'aniline utilisée contient au total 0,0001 à 0,4 % en poids, par rapport au poids de l'aniline utilisée, de composés qui contiennent au moins un groupe carbonyle ou qui sont formés par réaction de ces composés contenant au moins un groupe carbonyle avec de l' aniline .

3. Procédé selon la revendication 1, selon lequel l'aniline utilisée contient au total moins de 0,4 % en poids, par rapport au poids de l'aniline utilisée, de cétones (cyclo)aliphatiques éventuellement insaturées et/ou substituées.

4. Procédé selon la revendication 3, selon lequel le composé contenant des groupes carbonyle est la cyclohexanone.

5. Procédé selon la revendication 1, selon lequel l'aniline utilisée contient au total moins de 0,4 % en poids, par rapport au poids de l'aniline utilisée, de bases de Schiff.

6. Procédé selon la revendication 5, selon lequel la base de Schiff est la N-phénylcyclohexylimine.

7. Procédé de fabrication de di- et polyisocyanates de la série du diphénylméthane, selon lequel des di- et polyamines de la série du diphénylméthane sont fabriquées par le procédé selon l'une quelconque des revendications 1 à 6, puis mises en réaction avec du phosgène pour former les di- et polyisocyanates de la série du diphénylméthane.
